# EUROPEAN PATENT APPLICATION

(11) **EP 4 046 632 A1**
(43) Date of publication of application: **24.08.2022**
(21) Application number: 21382151.5
(22) Date of filing: 23.02.2021
(51) Int. Cl.: A61K 31/095, A61K 31/10, A61K 31/185, A61P 35/00, A61P 35/02

(54) **PROPYL PROPANE THIOSULFONATE FOR USE IN THE TREATMENT OF CANCER**

(71) Applicant: DMC Research Center, S.L., 18620 Alhendin Granada (ES)
(72) Inventor: Baños Arjona, Alberto, 18620 Alhandin (Granada) (ES); Guillamón Ayala, Enrique, 18620 Alhandin (Granada) (ES); Mut Salud, Nuria, 18620 Alhandin (Granada) (ES); Garrido Jiménez, José Manuela, 18620 Alhandin (Granada) (ES); Osuna Carrillo de Albornoz, Antonio, 18620 Alhandin (Granada) (ES); Rodríguez Serrano, Fernando, 18620 Alhandin (Granada) (ES); Maroto Cava, Francisco, 18620 Alhandin (Granada) (ES)
(74) Representative: Sahuquillo Huerta, Jesús

(57) **Abstract**

The present invention relates a method for the use of a product with propyl propane thiosulfonate (PTSO), whose purpose is the treatment and/or prophylaxis of malignant neoplasms such as solid cancers and myeloproliferative syndromes in humans. In a preferred embodiment, the method includes the selection of the PTSO compound which general formula is R-SOa-S-R, where "R" represents the n-propyl group (-CH₂-CH₂-CH₃) and "a" is 2.

## Description

### FIELD OF THE INVENTION

The present invention relates to the medical field, more concretely, the present invention relates to the antitumoral activity of propyl propane thiosulfonate (PTSO) compounds against malignant neoplasms or tumors, such as breast, lung, colorectal, pancreas and/or leukemia.

### BACKGROUND OF THE INVENTION

The term cancer includes all diseases characterized by the abnormal development of malignant cells, capable of multiplying without control, invading any organ or body tissue (Robles-Fernández et al. 2013). In normal tissues, there is a balance between the generation of new cells by cell division and the loss of these, through programmed cell death or apoptosis. However, tumor cells can evade the apoptosis process, continually dividing to form neoplasms or tumors that destroy and replace healthy tissues (Hanahan and Weinberg 2011).

Currently, cancer is one of the leading causes of morbidity and mortality worldwide, having caused 18.1 million new cases and 9.6 million deaths in 2018 (Bray et al. 2018). The treatment of these diseases should be conducted from a multidisciplinary approach, which includes surgical procedures, radiotherapy, and/or the use of chemotherapeutic agents. The main problem of conventional chemotherapy are the side effects it produces and that limits its application, in medium and long term. For this reason, the search for natural compounds that, in addition to being effective, cause a lower toxicity in the organism (Bishayee and Sethi 2016) (Bonofiglio et al. 2016) (Guardia et al. 2017) is still booming.

In the document EP3112346A1 it is disclose the use of PTSO for modulating immunological responses in humans. The PTSO is also known as propyl propane thiosulfonate, dipropylthiosulfonate or 1-propylsulfonisulfanylpropane (CAS number 1113-13-9). In this document it is generally disclosed the use of a PTSO product (a compound based on PTSO) that can be administered to improve humans Immune System to treat and/or prevent chronic infectious diseases, immunodeficiency disorders, tumors, a number of inflammatory bowel diseases, as well as other chronic autoimmune or inflammatory conditions or diseases. In the aforementioned document, the same inventor team reported the immunomodulatory properties of PTSO in different human cell lines, claiming the ability of this compound to inhibit or enhance the production of certain cytokines.

Document WO2008/077453A1 relates to 5-fluoropyrimidines compounds including the use of an organic propyl thiosulfonate whose main biological activity is in the cancer area. In this document multiple functional groups and compounds that can be part of one of the side chains (W) of 5-fluoropyrimidines compounds are mentioned, although in no case is there an intrinsic relationship between these side chains and the antitumor activity of their base compound which is what they really claim as anti-tumor. Furthermore, in the examples of his invention, the antitumor capacity has only been evaluated in two colon and ovarian lines with respect to the compounds 5-fluoro-2,4-dioxo-3,4-dihydropyrimidin-1 (2H) -yl) methyl 3 - (methylsulfonylthio)propanoate and 2- (methylsulfonylthio) ethyl 2-(5-fluoro-2,4-dioso-1,2,3,4-tetrahydropyrimidine-1-carbocamido) acetate. These compounds are structurally much more complex and totally differ from the properties of PTSO, furthermore the inventors have not provided evidence on the cytotoxicity of these compounds in healthy cells. Our invention represents a remarkable advance because, in addition to being a simpler compound, PTSO has demonstrated significant antitumor activity in different human cell lines (breast, colon, lung, pancreas, leukemia) while being safe for healthy cells, which it translates into a high therapeutic index.

Document WO2009/065926A1 relates to new compounds that are inhibitors of histone deacetylase (HDAC) and that release hydrogen sulfide, whose main biological activity is in the field of cancer and inflammation, the compound including the use of an organic propyl thiosulfonate. These inventors cover a wide spectrum of polysulfur compounds that contain two or more sulfide atoms in one of their radicals. That is, there is no direct relationship between the nature of this radical and the possible antitumor activity linked to the union of other structures described as X-Y-B. Furthermore, this document includes in its examples the potential antitumor activity in two lung lines without specifying the radicals involved, in addition to not evaluating their cytotoxic effect in healthy cells. It should be noted that these molecules are much more complex and release H₂S (a corrosive, toxic and flammable compound). That is, they totally differ in the properties of PTSO, a simpler, more stable molecule that does not release H₂S.

Finally, unlike other antitumor compounds, the low toxicity and safety of PTSO in rats has been demonstrated according the OECD guideline 408 no-observed-adverse-effect level (NOAEL) of PTSO was judged to be ≥ 55 mg/kg/day for both (Lira et al., 2020).

### SUMMARY OF THE INVENTION

The object of the present invention is the use of a PTSO compound as antitumoral. The antitumoral compound of the invention can be used to manufacture nutritional and pharmaceutical products for the treatment and/or prophylaxis of against malignant neoplasms, such as breast, lung, colorectal or pancreas cancer, leukemia and other acute and chronic proliferative syndromes in both animals and humans. The object of the invention is solved with the antitumoral compound of the claim 1. In the dependent claims it is disclosed other embodiments of the invention.

The scope of the present invention is defined by the claims. Throughout the description and claims, the word "comprises", and variants thereof do not seek to exclude other technical features, components, or steps. For those skilled in the art, other objects, advantages, and features of the invention will be inferred in part from the description and in part from practicing the invention.

### BRIEF DESCRIPTION OF THE DRAWINGS

A series of graphics which help to better understand the invention and are expressly related to an embodiment of said invention, taken as a non-limiting example thereof, are very briefly described below.
Figure 1 illustrates the examples of efficacy of PTSO in different human tumor lines (MCF-7, A-549, T-84, HT-29, Panc-1, and Jurkat)
Figure 2 illustrates the low cytotoxic effect of PTSO in normal human peripheral blood mononuclear cells (PBMCs)

### DETAILED DESCRIPTION OF THE INVENTION AND EXAMPLES

The present invention describes a novel method for the application of PTSO, which formula is R-SOa-S-R wherein "R" represents the n-propyl group (-CH₂-CH₂-CH₃) and "a" is 2. The present invention is applied to the treatment and/or prophylaxis of cancer, malignancies and myeloproliferative conditions including breast, lung, colon, pancreas, colon, lymphomas, and leukemia. In addition, PTSO can be administered to healthy humans, to prevent, treat and/or mitigate the effects of cancer, malignant tumors, and other myeloproliferative conditions.

It should be mentioned that, as set forth in the present invention, PTSO can be an active compound by itself and/or be combined with other antitumor compounds and/or pharmacological excipients. Therefore, it should be noted that the product with PTSO can be administered by itself (only PTSO, without other additives) or in combination with other compounds, active principles, and/or antitumor agents.

In an application form of the invention, the dose may be between 1 and 55 mg/kg bw/day. These concentrations have been established based on the effective dose obtained for each tumor line and the safe concentration at which PTSO does not produce toxicity in experimental animals.

In an embodiment of the invention, the use of a pharmacological composition containing PTSO as an active ingredient for the manufacture of an antitumor product is provided, which purpose is the treatment and/or prophylaxis of cancer and other myeloproliferative conditions.

Pharmacological compositions may include other drugs or antitumor compounds, as well as pharmaceutical excipients. In this case, it can be chosen the way to administer the product based on PTSO, being able to be orally, intravenously, topically, subdermally or rectally.

In another embodiment of the invention, there is provided the use of a nutritional composition containing PTSO as an active ingredient for the manufacture of an antitumor product whose purpose is the treatment and/or prophylaxis of cancer and other myeloproliferative conditions. Moreover, it is possible to incorporate PTSO into different products such as food, and/or nutritional supplements.

The application of the invention for oral administration can be conducted in the form of capsules, pills, tablets, powders, granules, as an emulsion or a liquid solution (drink or syrup). Additionally, PTSO can be administered by injection individually or as part of a pharmacological formulation.

The PTSO and therefore, any product based on PTSO, may be administered parenterally or orally, forming part of a pharmacological formulation, by incorporating it into a product and or food supplement, or by incorporating it into a nutritional supplement.

Evaluation of the antitumor activity *in vitro* of PTSO against human breast, lung, colon, pancreas and blood tumor cells.

### A) Cell lines

MCF-7 (Reference HTB-22 of the American Type Culture Collection): It is an epithelial-type tumor line derived from an adenocarcinoma of the human mammary gland of a 69-year-old Caucasian patient. These cells express estrogen receptors and the WNT7B oncogene, and among the cellular products they produce are the insulin-like factor binding proteins (IGFBP): BP-2, BP-4, and BP-5.

A-549 (Reference CCL-185 of the American Type Culture Collection): It is a tumor line of basal alveolar epithelium that comes from a pulmonary adenocarcinoma removed from a 58-year-old Caucasian patient. These cells are squamous and can synthesize lecithin.
T-84 (Reference CCL-248 of the American Type Culture Collection): It is a tumor line with epithelial morphology, isolated from a pulmonary metastasis of a colorectal carcinoma of a 72-year-old patient. These cells present receptors for a large group of hormones and neurotransmitters, and they produce carcinoembryonic antigen (CEA) and keratin, among other cellular products.

HT-29 (HTB-38 of the American Type Culture Collection): It is a human colon cell line with epithelial morphology, isolated from a colorectal adenocarcinoma of a 44-years-old Caucasian female patient.

Panc-1 (CRL-1469 of the American Type Culture Collection): It is a human tumor pancreatic cell line with epithelial morphology, isolated from a pancreatic carcinoma of a 56-years-old male patient.

Jurkat (Reference TIB-152 of the American Type Culture Collection): It is a T lymphocytes tumor line, from peripheral blood of a 14-year-old male patient with acute lymphoid leukemia. These cells could produce interleukin 2.

Peripheral blood mononuclear cells (PBMCs): These cells are lymphocytes and monocytes from normal or healthy blood cells, characterized by presenting a round nucleus.

All lines were provided by the Department of Cell Culture of the Center for Scientific Instrumentation of the University of Granada (Granada, Spain), and blood samples from healthy volunteers were supplied by the Biobank of the Public Health System of Andalusia.

### B) Maintenance of cultures and cell count.

The cells were grown in an oven at 37°C, with 5% CO₂ and 90% humidity, in a culture medium consisting of Dulbecco's Modified Eagle Medium (DMEM), supplemented with 10% heat-inactivated bovine fetal serum, 10 ml/L 100x penicillin-streptomycin and 2 mM L-glutamine. Cell cultures were maintained in sterile Falcon culture bottles (75 cm²). Culture mediums and the rest of supplements were supplied by Sigma-Aldrich (Sigma, St Louis, MO, USA). Cultures manipulation was carried out under sterile conditions, in a laminar flow cabinet (Aura Vertical S.D. 4 Bio Air Instruments, Italy). Once cultured cells reached a confluence of over 80%, these cells were divided and cultured in new culture flasks. This step was conducted, first discarding the culture medium, and gently washing the culture surface twice with sterile PBS. Next, 0.1 ml/cm² of trypsin, previously heated at 37°C, was added, and the culture flask was incubated for 4-5 minutes in an incubator at 37°C with 5% CO₂ to achieve cell detachment.

The trypsinization process was stopped by adding 0.1-0.2 ml/cm2 of complete culture medium (which includes FBS) to the cell aggregates, and then the cell solution was resuspended several times inside the culture flask with the help of a sterile pipette. The content of the culture flask was collected and passed to a sterile tube, which was centrifuged at 1500-1600 rpm for 5 minutes. After centrifugation, the tube was taken back to the flow cabinet, the supernatant was discarded, and the cell button was resuspended in few milliliters of complete culture medium. Then, cell count was performed using the Neubauer chamber, getting a concentration of 0.5 x 10⁶ cells/ml for a correct visualization and counting with the optic microscope.

### C) Methods of freezing and thawing cells.

The culture cells were separated with trypsin with the method explained above and stored under sterile conditions, in 2 ml cryotubes, with freezing medium consisting of FBS (fetal bovine serum) and DMSO (dimethyl sulfoxide) in 9: 1 ratio, first freezing at -80°C, and after 24-48 hours taking them to liquid nitrogen tanks at -176°C, to obtain a gradual freezing.

To thaw cells, the cryotubes were put with moist heat at 37°C, and quickly in a flow cabinet, the contents of cryotubes were resuspended in sterile tubes with complete culture medium at 37°C, then the tube was centrifuged for 5 minutes and 1500 rpm. Again, under sterile conditions, the cell button was resuspended in few milliliters of complete culture medium and passed into culture flasks at a concentration of 2x10⁴ cells/ml to start the culture process again.

### D) Synthesis and conservation of PTSO

The biosynthesis of PTSO is made from propiine (S-propyl-L-cysteine sulfoxide), an amino acid derived from the L-cysteine found in Allium species. The first step of biosynthesis is the formation of sulfenic acid (in this case, propyl-1-sulfenic acid; CH₃-CH₂-CH₂-SOH) in addition to pyruvate and ammonia.

However, these sulphonic acids are highly reactive, so they immediately produce thiosulphinates, the propylpropane thiosulphinate (PTS), by a condensation reaction. The oxidation of PTS induces its dismutation in PTSO and propyl disulfide. Propyl disulfide can also be oxidized and transformed into PTSO, and thus, the oxidation of PTS to PTSO is completed.

The PTSO was dissolved in DMSO and stored in aliquots at -20°C until use. The stock solution was diluted in culture medium to obtain the different concentrations required for each experiment, always resulting in a final DMSO concentration of less than 0.2% v/v.

### E) Treatment of cell lines with PTSO

Adherent cells (MCF-7, A-549, T-84, HT-29 and Panc-1), were detached from the surface of the culture flasks, using 3-4 ml of trypsin 1x, and left to act for 5-10 minutes in an incubator at 37°C with 5% CO₂. Once the cells were detached, the trypsinization was stopped, adding to the medium flask the same amount of complete culture as trypsin used to detach cells. After resuspending the mixture of trypsin and detached cells several times, the content of the culture flask was transferred to a sterile tube to centrifugation it for 5 minutes at 1500-1600 rpm.

In the case of non-adherent cells (Jurkat and PBMCs), the trypsinization step was obviated, centrifuging directly the cells contained in culture flasks. After centrifugation, the tube was taken to the flow cabinet, the supernatant was removed, and the cell button was resuspended in 2-3 ml of complete culture medium. After performing the cell count with the method described previously, cells were seeded in sterile plates of 24 or 96 wells, in a suitable volume of complete medium and stored in an incubator at 37°C with 5% CO₂.

Adherent cells were incubated for 24 hours to get cells attached to the surface of the flask. Then, the culture medium was replaced for a new medium, to which increasing concentrations of PTSO had been added, and the induction was conducted for 72 hours. In the case of non-adherent cells, the induction was conducted after seeding.
For each cell line studied, there were controls, where only complete culture medium was added, along with the equivalent amount of solvent (in this case, DMSO).

### F) Cytotoxicity assays in adherent cells (MCF-7, A-549, T-84, HT-29 and Panc-1).

The effect of increasing concentrations of PTSO on breast tumor cells (MCF-7), lung tumor (A-549), colon tumor (T-84 and HT-29) and pancreatic tumor (Panc-1) were evaluated.

Cells quantification was performed using a colorimetric technique with Sulforrodamine-B (SRB) (Vichai and Kirtikara 2006), as described below.

After the induction, the supernatant from wells was discarded and 300 µl of 10% trichloroacetic acid (TCA) was gently added at 4°C on the cell monolayer of each well, to get its fixing for 20 minutes in a refrigerator. Then, the supernatants were discarded, wells were washed with distilled water and finally the plates dried completely at room temperature.

Cells were stained by adding to each well 500 µl of SRB 0.4% and, after incubating at room temperature for 20-30 minutes with gentle agitation and darkness, the dye was discarded. Then, cells were three times washed with acetic acid 1%, and plates dried completely in darkness.

To solubilize the dye, 500 µl of Tris base pH: 10.5 was added to each well, and after 5 minutes of gentle agitation, aliquots of 100 µl were transferred to 96-well plates, which were read by a plate reader Titertek multiscan (Thermo Labsystems, USA) at 492 nm. The assessment of the optical density or absorbance was obtained using the "Ascent Software for Multiscan" program for Windows v.2.6 (Thermo Labsystems, USA).

Once the optical density values were obtained, a regression analysis was carried out for each cell line independently to establish the mathematical function that related the value of the number of cells with the obtained optical densities, using the Statgraphics program for Windows (Statistical Graphics Corp, 2000).

### G) Cytotoxicity assays in non-adherent cells (Jurkat and PBMCs).

The effect of increasing concentrations of PTSO on tumor lymphocytes (Jurkat) and PBMCs isolated from blood samples of healthy volunteers was evaluated. Cells were cultured in 96-well plates and induced by octuplicate with increasing concentrations of PTSO for 3 days. Induced cells were quantified by the MTT assay. First, cells were seeded at a concentration of 5 x 10⁴ cells/ml in 96-well plates, specifically 100 µl of cell suspension in each well. Immediately, PTSO was added in increasing concentrations to initiate induction.

After 72 hours, 20 µl of MTT were added to each well and the content of each well were resuspended several times. Then, the plate was incubated at 37°C for 3 hours to form formazan crystals. Several wells were left with only MTT, and others without PTSO as controls. After incubation, 100 µl of DMSO were added to each well and resuspended several times until the formazan crystals were dissolved. The 96-well plates were incubated in darkness for 1 hour, in gently agitation to facilitate the dissolution of the crystals, and finally were taken to a Titertek Multiskan plate reader (Thermo Labsystems, USA) to determine their optical density at 570 nm. The evaluation of the optical density was obtained using the "Ascent Software for Multiscan" program for Windows v.2.6 (Thermo Labsystems, USA).

Once the optical density values were obtained, a regression analysis was carried out for each cell line independently to establish the mathematical function that related the value of the number of cells with the obtained optical densities, using the Stat graphics program for Windows (Statistical Graphics Corp, 2000).

### H) Calculation of the IC₅₀ value and therapeutic index of PTSO.

The calculation of IC₅₀ was made from the semi-logarithmic dose-response curve by linear interpolation. Once all IC₅₀ values were obtained, the therapeutic index (IT) was determined, dividing the IC₅₀ value of PTSO in the normal line by the IC₅₀ value of a tumor line.

The results show that PTSO exerted antitumor activity against MCF-7 (human breast adenocarcinoma line), A-549 (human lung adenocarcinoma line), T-84 (human colorectal adenocarcinoma line), HT-29 (human colorectal carcinoma line), Panc-1 (human pancreatic carcinoma line) and Jurkat (human tumor T lymphocytes line), with IC₅₀ values of 6.9, 38.6, 37.3, 50.8, 105.2 and 15.7 µM, respectively, as can be shown in Table 1, wherein the IC₅₀ values expressed as means ± SEM of six independent determinations in the µM range.

**Table 1**

| **Cell Line** | **IC₅₀ (µM)** |
|---|---|
| MCF-7 | 6.9 ± 0.1 |
| A-549 | 38.6 ± 0.4 |
| T-84 | 37.3 ± 0.8 |
| HT-29 | 50.8 ± 3.7 |
| Panc-1 | 105.2 ± 4.2 |
| Jurkat | 15.7 ± 0.5 |
| PBMCs | 248.5 ± 46 |

Considering the value of IC₅₀ on normal PBMCs cells (248.5 µM), the *in vitro* T.I (IC₅₀ Normal Line/IC₅₀ Tumor Line) of PTSO is 36.0 taking as reference the MCF-7 tumor line, and 15.8 taking as reference the Jurkat tumor line. The higher the T.I of a drug or compound, the lower its toxicity in normal healthy cells. Therefore, our results would indicate the usefulness of PTSO for the establishment of treatments against neoplastic pathologies, with a safety range that avoids the appearance of toxicity effects, since the dose of PTSO capable of eliminating the 50% of different tumor lines populations, was innocuous for healthy PBMCs.

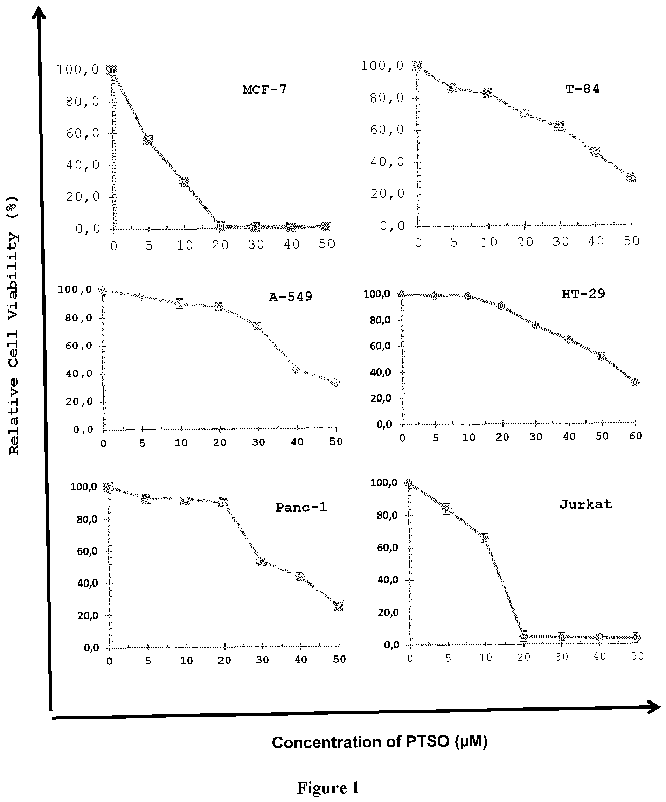

Figure 1 illustrates graphically the antiproliferative activity of PTSO with the dose-response curves of cultures of MCF-7, A-549, T-84, HT-29, Panc-1 and Jurkat cells, induced for 3 days with increasing concentrations of PTSO.

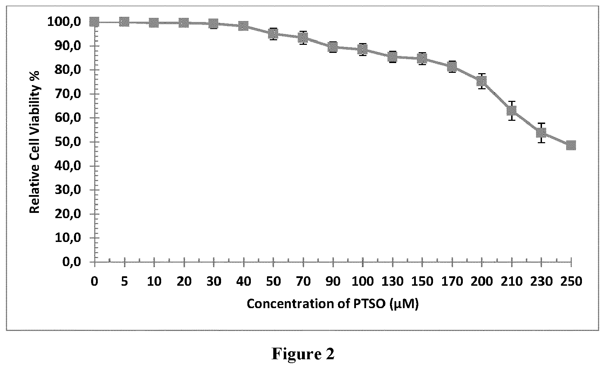

Figure 2 illustrates graphically the low cytotoxic activity of PTSO with the dose-response curves of culture of normal human PBMCs, induced for 3 days with increasing concentrations of PTSO.

### Citation list

Bishayee, Anupam, and Gautam Sethi. 2016. 'Bioactive Natural Products in Cancer Prevention and Therapy: Progress and Promise'. Seminars in Cancer Biology 40-41: 1-3.

Bonofiglio, Daniela, Cinzia Giordano, Francesca De Amicis, Marilena Lanzino, and Sebastiano Ando. 2016. 'Natural Products as Promising Antitumoral Agents in Breast Cancer: Mechanisms of Action and Molecular Targets'. Mini Reviews in Medicinal Chemistry 16 (8): 596-604.

Bray, Freddie, Jacques Ferlay, Isabelle Soerjomataram, Rebecca L. Siegel, Lindsey A. Torre, and Ahmedin Jemal. 2018. 'Global Cancer Statistics 2018: GLOBOCAN Estimates of Incidence and Mortality Worldwide for 36 Cancers in 185 Countries'. CA: A Cancer Journal for Clinicians 68 (6): 394-424.

Guardia, Juan J., Rubén Tapia, Soumicha Mahdjour, Fernando Rodriguez-Serrano, Nuria Mut-Salud, Rachid Chahboun, and Enrique Alvarez-Manzaneda. 2017. 'Antiproliferative Activity of Natural Taiwaniaquinoids and Related Compounds'. Journal of Natural Products 80 (2): 308-18.

Hanahan, Douglas, and Robert A. Weinberg. 2017. 'Biological Hallmarks of Cancer'. In Holland-Frei Cancer Medicine, 1-10. American Cancer Society.

Lira AC, Prieto AI, Baños A, Guillamon E, Moyano R, Jos A, Cameán AM. Safety assessment of propyl-propane-thiosulfonate (PTSO): 90-days oral subchronic toxicity study in rats. Food Chem Toxicol. 2020 Oct; 144:111612. doi: 10.1016/j.fct.2020.111612. Epub 2020 Jul 29. PMID: 32738370.

Vichai, Vanicha, and Kanyawim Kirtikara. 2006. 'Sulforhodamine B Colorimetric Assay for Cytotoxicity Screening'. Nature Protocols 1 (3): 1112-16. https://doi.org/10.1038/nprot.2006.179.

## Claims

1. An antitumoral formulation comprising as an active ingredient a PTSO compound represented by the formula R-SOa-S-R wherein R represents a n-propyl group (-CH₂-CH₂-CH₃) and a is 2.

2. A pharmaceutical composition for preventing and/or treating tumors comprising the antitumoral formulation of claim 1 and one or more pharmacologically acceptable carriers and excipients.

3. The pharmaceutical composition of claim 2 wherein the tumors include breast cancer, lung cancer, colorectal cancer, pancreas cancer and/or leukemia.

4. A combination of the antitumoral formulation of claim 1 and one or more other compounds having an antitumor effect for use in the prevention and/or treatment of tumors.

5. The combination of claim 4 wherein the tumors include breast cancer, lung cancer, colorectal cancer, pancreas cancer and/or leukemia.

6. A nutritional supplement comprising the antitumoral formulation of claim 1 for use in the prevention and/or treatment of tumors.

7. The nutritional supplement of claim 6 wherein the tumors include breast cancer, lung cancer, colorectal cancer, pancreas cancer and/or leukemia.
